# EUROPÄISCHE PATENTSCHRIFT

(11) **EP 0 767 769 B1**
(45) Veröffentlichungstag und Bekanntmachung des Hinweises auf die Patenterteilung: **07.10.1998**
(21) Anmeldenummer: 95923336.2
(22) Anmeldetag: 16.06.1995
(51) Int. Cl.: C07C 29/141, C07C 31/20, C07C 45/59, C07C 47/19, C07D 307/20

(54) **VERFAHREN ZUR HERSTELLUNG VON 1,4-BUTANDIOL**
METHOD OF PRODUCING 1,4-BUTANEDIOL
PROCEDE DE PREPARATION DE 1,4-BUTANEDIOL

(30) Priorität: 27.06.1994 DE 4422051
(43) Veröffentlichungstag der Anmeldung: 16.04.1997
(73) Patentinhaber: BASF AKTIENGESELLSCHAFT, 67056 Ludwigshafen (DE)
(72) Erfinder: PINKOS, Rolf, D-67098 Bad Dürkheim (DE); FISCHER, Rolf, D-69121 Heidelberg (DE); BREITSCHEIDEL, Boris, D-36043 Fulda (DE); POLANEK, Peter, D-69469 Weinheim (DE)
(86) Internationale Anmeldenummer: EP9502335
(87) Internationale Veröffentlichungsnummer: WO9600203

(56) Entgegenhaltungen:
- WO-A-92/20667
- US-A- 4 476 332
- US-A- 4 859 801
- TETRAHEDRON LETTERS, Bd. 33, Nr. 42, 13.Oktober 1992 OXFORD, GB, Seiten 6371-6374, S. BASKARAN, ET AL.: 'A novel and unusual reaction of enol ethers with benzyltriethylammonium borohydride and chlorotrimethylsilane'

## Beschreibung

Die vorliegende Erfindung betrifft ein Verfahren zur Herstellung von 1,4-Butandiol.

WO 92/20667 betrifft ein Verfahren zur Herstellung von 4-Hydroxybutyraldehyd/2-Hydroxytetrahydrofuran-Gemischen, in dem zunächst in einer ersten Stufe 2,5-Dihydrofuran in Gegenwart homogen im Reaktionsmedium gelöster Rhodium- oder Ruthenium-Phosphin-Komplexe das 2,5-Dihydrofuran zu 2,3-Dihydrofuran isomerisiert und anschließend aus der Reaktionsmischung abdestilliert wird. Sodann wird das 2,3-Dihydrofuran an einem aciden Katalysator mit Wasser zu einem Gemisch aus 4-Hydroxybutanal und 2-Hydroxytetrahydrofuran umgesetzt, und dieses Gemisch isoliert. Es wird vorgeschlagen, das so erhaltene Gemisch durch Hydrierung in 1,4-Butandiol umzuwandeln. Ausgehend von 2,3-Dihydrofuran werden somit 2 Stufen benötigt, um zu 1,4-Butandiol zu gelangen.

In US-A 4 859 801 wird gelehrt, 2,3-Dihydrofuran in Gegenwart von Wasser bei einem pH-Wert von 8 bis 14 mit einem Aldehyd und Wasserstoff mittels eines Hydrierkatalysators zu Gemischen aus 1,4-Butandiol und 2-Alkyl-1,4-butandiol umzusetzen. Die Ausbeute an 1,4-Butandiol ist dabei bescheiden. Soweit das in die Umsetzung eingesetzte 2,3-Dihydrofuran direkt hydriert wird, entsteht daraus als Hauptprodukt Tetrahydrofuran. 1,4-Butandiol wird nur in geringen Mengen gebildet.

EP-A 340 970 betrifft die Hydrierung vorher separat hergestellter 4-Hydroxybutyraldehyd/2 -Hydroxytetrahydrofuran-Gemische in basischem Medium und in Gegenwart eines Aldehyds zu 2-Alkyl-1,4-butandiolen, wobei ein Teil dieses Gemisches zu 1,4-Butandiol hydriert wird.

Der vorliegenden Erfindung lag daher die Aufgabe zugrunde, ein Verfahren zur Herstellung von 1,4-Butandiol ausgehend von 2,3-Dihydrofuran zu finden, das es ermöglicht, 1,4-Butandiol einstufig in guter Ausbeute und Selektivität zu erhalten.

Dementsprechend wurde ein Verfahren zur Herstellung von 1,4-Butandiol gefunden, das dadurch gekennzeichnet ist, daß man 2,3-Dihydrofuran einstufig, in Gegenwart von Wasser und Wasserstoff bei einer Temperatur von 20 bis 300°C und einem Druck von 1 bis 300 bar in neutraler oder saurer Umgebung an einem Hydrierkatalysator umsetzt.

In dem erfindungsgemäßen Verfahren werden somit die 2 Reaktionsstufen a) Umsetzung des 2,3-Dihydrofuran mit Wasser zu einem Gemisch aus 4-Hydroxybutyraldehyd und dessen Isomerem 2-Hydroxytetrahydrofuran gemäß Gleichung (1) 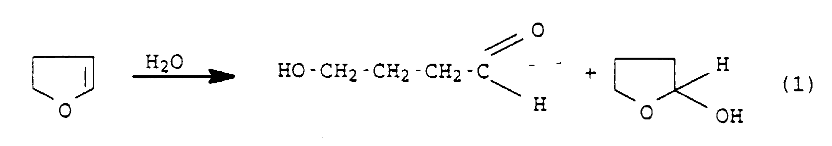 und b) die katalytische Hydrierung des gemäß Gleichung (1) erhaltenen Gemisches aus 4-Hydroxybutyraldehyd und 2-Hydroxytetrahydrofuran - beide Verbindungen stehen miteinander im Gleichgewicht - zu 1,4-Butandiol gemäß Gleichung (2) 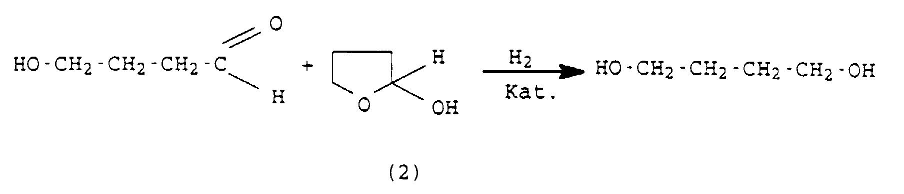 in einer einzigen Verfahrensstufe bewirkt.

Bei der Durchführung des erfindungsgemäßen Verfahrens wird das 2,3-Dihydrofuran mit Wasser im allgemeinen in einem Molverhältnis 2,3-Dihydrofuran/Wasser von 1:1 bis 1:100, vorzugsweise von 1:1 bis 1:50 und besonders bevorzugt von 1:1 bis 1:10 und in Gegenwart von Wasserstoff und eines Hydrierkatalysators bei einem Druck von im allgemeinen 1 bis 300 bar, vorzugsweise von 5 bis 250 bar und insbesondere von im allgemeinen 15 bis 200 bar und bei einer Temperatur von 20 bis 300°C, vorzugsweise von 40 bis 230°C und besonders bevorzugt von 80 bis 200°C zu 1,4-Butandiol umgesetzt.

Die erfindungsgemäße Umsetzung wird in neutraler oder saurer Umgebung durchgeführt, d.h. bei pH-Werten der wäßrigen Phase, die im sauren oder neutralen pH-Bereich liegen, vorzugsweise bei pH-Werten im Bereich von 2 bis 7,5, insbesondere von 4 bis 7,2 und, besonders bevorzugt im Bereich von 6 bis 7. Bei Verwendung wasserunlöslicher heterogener Katalysatoren bedeutet das Arbeiten in neutraler oder saurer Umgebung, daß die verwendeten Katalysatoren nicht basisch wirken und vorzugsweise Lewis- oder Brönsted-acide Zentren enthalten, die den Verlauf der Umsetzung im gewünschten Sinne, nämlich der überwiegenden Bildung von 1,4-Butandiol, beeinflussen.

Als Hydrierkatalysatoren können im erfindungsgemäßen Verfahren im allgemeinen alle zur Hydrierung von Carbonylgruppen geeigneten Katalysatoren Verwendung finden. Es können sowohl homogen im Reaktionsmedium lösliche Hydrierkatalysatoren, wie sie beispielsweise in Houben-Weyl, Methoden der Organischen Chemie, Band IV/1c, S. 45 bis 67, Thieme Verlag, Stuttgart 1980 beschrieben werden oder aber auch heterogene Hydrierkatalysatoren, wie sie in Houben-Weyl, Methoden der Organischen Chemie, Band IV/1c, S. 16 bis 26 beschrieben werden, benutzt werden. Bevorzugte Homogenkatalysatoren sind insbesondere die Komplexe des Rhodiums, Rutheniums und Kobalts mit Phosphin- oder Phosphit-Liganden, deren Herstellung z.B. in CA-A 7 276 41, H. Brunner in Hartley: The chemistry of the metal-carbon bond; Vol. 5, S. 110-124, John Wiley & Sons, New York 1989 sowie Tóth et al, Inorg. Chim. Acta 42, 153 (1980) und der dort zitierten Literatur beschrieben wird.

Vorzugsweise wird im erfindungsgemäßen Verfahren jedoch mit heterogenen Hydrierkatalysatoren gearbeitet, also solchen Hydrierkatalysatoren, die im Reaktionsmedium im wesentlichen unlöslich sind. Von diesen Hydrierkatalysatoren sind solche bevorzugt, die ein oder mehrere Elemente der Gruppe Ib, VIIb und VIIIb des Periodensystems der Elemente, insbesondere Kupfer, Rhenium oder Ruthenium oder Gemische dieser Elemente enthalten.

Bevorzugte Katalysatoren sind weiterhin solche, die mindestens ein Element der Gruppe Ib, VIIb oder VIIIb und noch mindestens ein weiteres Element aus den Gruppen IB, Vb, VIb, VIIb, VIIIb, IIIa oder IVa des Periodensystems der Elemente enthalten, das mit dem oder den Elementen der Gruppe Ib, VIIb oder VIIIb eine Mischung oder Legierung bildet. Außer den obengenannten Elementen Kupfer, Rhenium und Ruthenium, seien hierfür beispielhaft die Elemente Chrom, Molybdän, Wolfram, Kobalt, Rhodium, Iridium, Nickel, Palladium, Eisen und/oder Platin genannt.

Im erfindungsgemäßen Verfahren können heterogene Hydrierkatalysatoren verwendet werden, die aus Metallen in aktivierter, feinverteilter Form mit großer Oberfläche bestehen, beispielsweise Raney-Kupfer oder Rhenium-Schwamm.

Des weiteren können im erfindungsgemäßen Verfahren z.B. sogenannte Fällungskatalysatoren verwendet werden. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder -Carbonat-Lösungen, als z.B. schwerlösliche Hydroxyde, Oxydhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C in die betreffenden Oxide, Mischoxide und/oder gemischtvalentigen Oxide umwandelt, welche durch eine Behandlung mit Wasserstoff oder mit Wasserstoff enthaltenden Gasen bei in der Regel 100 bis 700°C, insbesondere bei 150 bis 400°C, zu den betreffenden Metallen und/oder oxidischen Verbindungen niederer Oxidationsstufe reduziert und in die eigentliche, katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den obengenannten Fällungskatalysatoren, welche außer den katalytisch aktiven Komponenten noch zusätzlich ein Trägermaterial enthalten, eignen sich für das erfindungsgemäße Verfahren im allgemeinen solche Trägerkatalysatoren, bei denen die katalytisch-hydrierend wirkenden Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diese Trägermaterialien z.B. durch Tränkung mit Lösungen oder Suspensionen der Salze oder Oxide der betreffenden Elemente, Trocknung und anschließender Reduktion der Metallverbindungen zu den betreffenden Metallen oder Verbindungen niederer Oxidationsstufe mittels eines Reduktionsmittels, vorzugsweise mit Hilfe von Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder mit thermisch leicht zersetzbaren Komplexverbindungen, z.B. mit Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfung oder durch Flammspritzen abgeschieden werden.

Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte dieser Trägerkatalysatoren an katalytisch aktiven Metaller zu höheren Raum-Zeit-Umsätzen führen als niedrigere Gehalte. Im allgemeinen werden aber Trägerkatalysatoren verwendet, deren Gehalt an katalytisch aktiven Metallen 0,1 bis 80 Gew.-%, vorzugsweise 0,5 bis 30 Gew.-% und bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach den Verfahren von DE-A 25 19 817, EP-A 1 477 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung auf einem abgeschiedenen Salze oder Komplexe der zuvor genannten Metalle, erzeugt werden.

Die Aktivierung der Fällungskatalysatoren als auch der Trägerkatalysatoren kann auch in situ in der Reaktionsmischung durch den dort anwesenden Wasserstoff erfolgen, bevorzugt werden diese Katalysatoren jedoch vor ihrer Verwendung separat aktiviert.

Als Trägermaterialien können im allgemeinen die Oxide des Aluminiums und Titans, Zirkoniumdioxid, Siliciumdioxid, Kieselgur, Kieselgel, Tonerden, z.B. Montmorillonite, Silikate, wie Magnesium- oder Aluminiumsilikate, Zeolithe, wie ZSM-5 oder ZSM-10-Zeolithe sowie Aktivkohle verwendet werden. Bevorzugte Trägermaterialien sind Aluminiumoxide, Titandioxide, Zirkoniumdioxid und Aktivkohle. Selbstverständlich können auch Mischungen verschiedener Trägermaterialien als Trager für im erfindungsgemäßen Verfahren anwendbare Katalysatoren dienen.

Als für im erfindungsgemäßen Verfahren einsetzbare Heterogenkatalysatoren seien die folgenden Katalysatoren beispielhaft genannt:

Mangan auf Aktivkohle, Rhenium auf Aktivkohle, Rhenium auf Siliciumdioxid, Rhenium/Zinn auf Aktivkohle, Rhenium/Palladium auf Aktivkohle, Rhenium/Kupfer auf Aktivkohle, Rhenium/Nickel auf Aktivkohle, Kupfer auf Aktivkohle, Kupfer auf Siliciumdioxid, Kupfer auf Aluminiumoxid, Kupferchromit sowie Bariumkupferchromit.

Den Katalysatoren konnen Lewis- und/oder Brönsted-acide Komponenten, wie Zeolithe, Aluminium- oder Siliciumoxide, Phosphorsäure oder Schwefelsäure, zugesetzt werden. Sie werden im allgemeinen in Mengen von 0,01 bis 5 Gew.-%, vorzugsweise von 0,05 bis 0,5 Gew.-% und besonders bevorzugt von 0,1 bis 0,4 Gew.-%, bezogen auf das Gewicht des eingesetzten Katalysators, zugesetzt.

Besonders bevorzugt wird das erfindungsgemäße Verfahren so ausgeführt, daß man Hydrierkatalysatoren verwendet, die Brönsted- und/ oder Lewis-acide Zentren enthalten. Bei der Verwendung derartiger Katalysatoren ist im allgemeinen die zusätzliche Zugabe einer Brönsted- oder Lewis-Säure zur Reaktionsmischung nicht erforderlich.

Als Brönsted-Säurezentren-haltige Homogenkatalysatoren können z.B. Übergangsmetallkomplexe von Metallen der Gruppe VIIIb, insbesondere Rhodium-, Ruthenium- und Kobalt-Komplexe mit Phosphin- oder Phosphit-Liganden verwendet werden, welche Brönsted-acide funktionelle Gruppen, wie Carboxyl-, Sulfonsäure- und/ oder Phosphonsäuregruppen als Substituenten tragen, beispielsweise Komplexe der genannten Übergangsmetalle mit Triphenylphosphin-p-sulfonsäure-Liganden. Solche Liganden können z.B. nach dem Verfahren von Angew. Chem. 105, 1097 (1993) hergestellt werden.

Besonders vorteilhafte Ergebnisse können im erfindungsgemäßen Verfahren mit Heterogenkatalysatoren, die Brönsted- oder Lewis-saure Zentren enthalten, erzielt werden. Als Brönsted- oder Lewis-saure Zentren können z.B. die katalytisch aktiven Metalle selber wirken, wenn diese bei der Aktivierung des Katalysators 5 mit Wasserstoff oder Wasserstoff-enthaltenden Gase nicht vollständig zu den betreffenden Metallen reduziert werden. Dies gilt z.B. für die Rhenium- und Chromit-haltigen Katalysatoren wie Rheniumschwarz und Kupferchromit. Im Rheniumschwarz liegt das Rhenium als Mischung von Rheniummetall mit Rheniumverbindungen in höheren Oxidationsstufen vor, wobei letztere Wirkungen wie Lewis- oder Brönsted-Säuren entfalten können. Weiterhin können 5 solche Lewis- oder Brönsted-aciden Zentren über das verwendete Trägermaterial in den Katalysator eingebracht werden. Als Lewis- oder Brönsted-acide Zentren enthaltende Trägermaterialien seien z.B. die Aluminiumoxide, Titandioxide, Zirkoniumdioxid, Siliciumdioxid, die Silikate, Tonerden, Zeolithe und Aktivkohle genannt.

Besonders bevorzugt werden deshalb im erfindungsgemäßen Verfahren als Hydrierkatalysatoren Trägerkatalysatoren verwendet, die mindestens ein Element aus der Gruppe Ib, VIIb oder VIIIb des Periodensystems der Elemente, insbesondere Kupfer, Rhenium und/oder Ruthenium oder die mindestens ein Element aus der Gruppe Ib, VIIb oder VIIIb und noch mindestens ein weiteres Element aus den Gruppen Ib, Vb, VIb, VIIb, VIIIb, IIIa oder IVa des Periodensystems der Elemente enthalten, das mit dem oder den Elementen der Gruppe Ib, VIIb oder VIIIb eine Mischung oder Legierung bildet, auf einem Brönsted- oder Lewis-acide wirkenden Trägermaterial abgeschieden enthalten. Besonders vorteilhafte Katalysatoren sind z.B. Rhenium auf Aktivkohle, Rhenium auf Zirkoniumdioxid, Rhenium auf Titandioxid, Rhenium auf Siliciumdioxid, Kupfer auf Aktivkohle, Kupfer auf Siliciumdioxid und Ruthenium auf Aktivkohle.

Das erfindungsgemäße Verfahren kann sowohl kontinuierlich als auch diskontinuierlich ausgeübt werden. Zur kontinuierlichen Betriebsweise können beispielsweise Rohrreaktoren vorteilhaft eingesetzt werden, in denen der Katalysator vorteilhaft in Form eines Festbetts angeordnet ist, über das die Reaktionsmischung in der Sumpf- oder Rieselfahrweise geleitet werden kann. Bei der diskontinuierlichen Betriebsweise können sowohl einfache Rührreaktoren oder vorteilhaft Schlaufenreaktoren verwendet werden. Bei Verwendung von Schlaufenreaktoren wird der Katalysator zweckmäßigerweise in Form eines Festbetts angeordnet. Bei nichtvollständiger Umsetzung des Ausgangsmaterials kann dieses zweckmäßigerweise entweder nach destillativer Abtrennung der Wertprodukte oder als Teilstrom zusammen mit den anderen Reaktionsprodukten in die Umsetzung zurückgeführt werden. Dies kann sich insbesondere bei der kontinuierlichen Betriebsweise als vorteilhaft erweisen. Im allgemeinen werden bei kontinuierlicher Reaktionsführung höhere Ausbeuten an 1,4-Butandiol erhalten als bei diskontinuierlicher Reaktionsführung am selben Katalysator.

Das erfindungsgemaße Verfahren kann vorteilhaft in Anwesenheit eines unter den Reaktionsbedingungen inerten Lösungsmittels durchgeführt werden, beispielsweise mit wasserlöslichen Ethern, wie Tetrahydrofuran, Dioxan oder Dimethoxymethan. Vorteilhaft können auch Alkohole, insbesondere das Verfahrensprodukt 1,4-Butandiol, als Lösungsmittel eingesetzt werden.

Als Reaktionsaustrag wird im allgemeinen ein Gemisch erhalten, das überwiegend aus überschüssigem Wasser und 1,4-Butandiol zusammengesetzt ist. Als Nebenprodukte können in geringen Mengen beispielsweise γ-Butyrolacton, Tetrahydrofuran und n-Butanol im Austrag enthalten sein. Der Reaktionsaustrag kann nach an sich herkömmlichen Verfahren, z.B. destillativ, zur Isolierung des 1,4-Butandiols und gewünschtenfalls der im Reaktionsaustrag enthaltenen Nebenprodukte γ-Butyrolacton, Tetrahydrofuran und n-Butanol, aufgearbeitet werden. Dabei kann gegebenenfalls nicht umgesetztes 2,3-Dihydrofuran sowie das gegebenenfalls verwendete Lösungsmittel zurückgewonnen und in die Umsetzung zurückgeführt werden. Bei unvollständigem Umsatz des 2,3-Dihydrofurans kann der Reaktionsaustrag vor seiner Aufarbeitung auch in einem Nachreaktor bis zum vollständigen Umsatz nachbehandelt werden.

Das als Ausgangsmaterial benötigte 2,3-Dihydrofuran ist z.B. nach dem Verfahren von US-A 3 828 077 durch die partielle Hydrierung von Furan erhältlich.

1,4-Butandiol wird weltweit in großem Umfang hergestellt und dient als Diolkomponente zur Herstellung von u.a. Polyestern, Polyurethanen und Epoxidharzen.

### Beispiele

Die Ausbeuten der folgenden Beispiele sind in Mol-% angegeben und wurden gaschromatographisch ermittelt.

### Beispiel 1

In einen 50 ml Metallautoklaven mit Rührer wurden 2 g eines bei 300°C im Wasserstoffstrom aktivierten Rhenium auf Aktivkohle-Katalysators mit einem Rhenium-Gehalt von 6 Gew.-%, berechnet als Re und bezogen auf das Gewicht des Katalysators, 5 g 2,3-Dihydrofuran und 15 g Wasser gefüllt. 50 bar Wasserstoff wurden aufgepreßt, dann wurde auf 170°C aufgeheizt. Nach 1 Stunde wurde abgekühlt und entspannt. Der Reaktionsaustrag hatte folgende 5 Zusammensetzung: 77 mol-% 1,4-Butandiol, 20 Mol-% γ-Butyrolacton, 1,3 Mol-% Tetrahydrofuran, 1,3 Mol-% n-Butanol und 0,3 Mol-% n-Propanol.

### Beispiel 2

Analog Beispiel 1 wurden 5 g 2,3-Dihydrofuran und 5 g Wasser an 2 g eines Kupfer auf Aktivkohle-Katalysators (Kupfergehalt: 10 Gew.-%, berechnet als Cu und bezogen auf das Gesamtgewicht des Katalysators; hergestellt durch Tränken der Aktivkohle mit der entsprechenden Menge einer Kupferammoniakatlösung, anschließender Trocknung bei 120°C und 2 stündiger Aktivierung im Wasserstoffstrom bei 300°C) zwei Stunden lang umgesetzt. Der Reaktionsaustrag hatte folgende Zusammensetzung: 95 Mol-% 1,4-Butandiol, 4 Mol-% γ-Butyrolacton und 0,8 Mol-% Tetrahydrofuran. Der Rest bestand überwiegend aus dem Acetal aus 2-Hydroxytetrahydrofuran und 1,4-Butandiol.

### Beispiel 3

In einem 25 ml fassenden Rohrreaktor wurden 25 ml des Katalysators gemäß Beispiel 1 eingebaut. Anschließend wurden 10 g 2,3-Dihydrofuran/h und 5 g Wasser/h über zwei getrennte Zuläufe in den Kopf des Reaktors geleitet. Der Wasserstoffdruck im Reaktor betrug 120 bar, die Temperatur 166°C. Das Reaktorabgas betrug 50 l/h. Im Reaktionsaustrag fanden sich bei 97 % Umsatz 80 Mol-% 1,4-Butandiol, 1,6 Mol-% Tetrahydrofuran, 8,3 Mol-% γ-Butyrolacton, 1,3 Mol-% n-Butanol. Der Rest bestand aus dem Acetal aus 2-Hydroxytetrahydrofuran und 1,4-Butandiol.

Der über 8 Stunden gesammelte Reaktionsaustrag wurde nach Beendigung des Versuchslaufs unter den gleichen Reaktionsbedingungen erneut über den selben Katalysator geleitet (ein Zulauf, 20 g/h. Bei vollständigem 2,3-Dihydrofuran-Umsatz ergaben sich folgende Ausbeuten: 92 Mol-% 1,4-Butandiol, 1,9 Mol-% Tetrahydrofuran, 4,1 Mol-% γ-Butyrolacton und 2 Mol-% n-Butanol.

## Patentansprüche

1. Verfahren zur Herstellung von 1,4-Butandiol, dadurch gekennzeichnet, daß man 2,3-Dihydrofuran einstufig, in Gegenwart von Wasser und Wasserstoff bei einer Temperatur von 20 bis 300°C und einem Druck von 1 bis 300 bar im pH-Bereich von 2 bis 7,5 an einem Hydrierkatalysator umsetzt.

2. Verfahren nach Anspruch 1, dadurch gekennzeichnet, daß man einen heterogenen Hydrierkatalysator einsetzt.

3. Verfahren nach den Ansprüchen 1 und 2, dadurch gekennzeichnet, daß man einen Hydrierkatalysator verwendet, der mindestens ein Element aus der Gruppe Ib oder VIIb oder VIIIb des Periodensystems der Elemente oder Gemische dieser Elemente enthält.

4. Verfahren nach den Ansprüchen 1 bis 3, dadurch gekennzeichnet, daß man einen Hydrierkatalysator verwendet, dessen katalytisch aktive Komponente auf einen Träger aufgebracht worden ist.

5. Verfahren nach den Ansprüchen 1 bis 4, dadurch gekennzeichnet, daß man einen Hydrierkatalysator verwendet, der eine oder mehrere Brönsted- oder Lewis-acide wirkende Komponenten enthält.

6. Verfahren nach den Ansprüchen 1 bis 5, dadurch gekennzeichnet, daß man einen Hydrierkatalysator verwendet, der Rhenium enthält.

7. Verfahren nach den Ansprüchen 1 bis 5, dadurch gekennzeichnet, daß man einen Hydrierkatalysator verwendet, der Kupfer enthält.

8. Verfahren nach den Ansprüchen 1 bis 5, dadurch gekennzeichnet, daß man einen Hydrierkatalysator verwendet, der Ruthenium enthält.

9. Verfahren nach den Ansprüchen 1 bis 7, dadurch gekennzeichnet, daß der Katalysator mindestens ein Element der Gruppe Ib, VIIb oder VIIIb und noch mindestens ein weiteres der Gruppe Ib, Vb, VIb, VIIb, VIIIb, IIIa und IVa des Periodensystems der Elemente enthält, das mit dem oder den Elementen der Gruppe Ib oder VIIb oder VIIIb eine Mischung oder eine Legierung bildet.

10. Verfahren nach den Ansprüchen 1 bis 8, dadurch gekennzeichnet, daß man einen Hydrierkatalysator verwendet, dessen katalytisch aktive Komponente auf einem Trägermaterial aufgebracht worden ist, aus Aluminiumoxid, Tonerden, Siliciumdioxid, Zirkoniumdioxid, Titandioxid, einen Zeolithen und/ oder Aktivkohle enthält.

11. Verfahren nach Anspruch 1, dadurch gekennzeichnet, daß man einen homogenen Hydrierkatalysator verwendet, der ein Element der Gruppe VIIIb des Periodensystems der Elemente enthält.

## Claims

1. A process for preparing 1,4-butanediol, which comprises reacting 2,3-dihydrofuran in one stage over a hydrogenation catalyst in the presence of water and hydrogen at from 20 to 300°C and a pressure of from 1 to 300 bar in a pH range from 2 to 7.5.

2. A process as claimed in claim 1, wherein a heterogeneous hydrogenation catalyst is used.

3. A process as claimed in claim 1 or 2, wherein the hydrogenation catalyst used comprises at least one element from group Ib or VIIb or VIIIb of the Periodic Table of the Elements or a mixture of these elements.

4. A process as claimed in any of claims 1 to 3, wherein use is made of a hydrogenation catalyst whose catalytically active component has been applied to a support.

5. A process as claimed in any of claims 1 to 4, wherein the hydrogenation catalyst used comprises one or more components which act as Brönsted or Lewis acids.

6. A process as claimed in any of claims 1 to 5, wherein the hydrogenation catalyst used comprises rhenium.

7. A process as claimed in any of claims 1 to 5, wherein the hydrogenation catalyst used comprises copper.

8. A process as claimed in any of claims 1 to 5, wherein the hydrogenation catalyst used comprises ruthenium.

9. A process as claimed in any of claims 1 to 7, wherein the catalyst comprises at least one element of group Ib, VIIb or VIIIb and additionally at least one further element of groups Ib, Vb, VIb, VIIb, VIIIb, IIIa and IVa of the Periodic Table of the Elements which forms a mixture or an alloy with the element(s) of group Ib or VIIb or VIIIb.

10. A process as claimed in any of claims 1 to 8, wherein use is made of a hydrogenation catalyst whose catalytically active component has been applied to a support material comprising aluminum oxide, aluminas, silicon dioxide, zirconium dioxide, titanium dioxide, a zeolite and/or activated carbon.

11. A process as claimed in claim 1, wherein use is made of a homogeneous hydrogenation catalyst which comprises an element of group VIIIb of the Periodic Table of the Elements.

## Revendications

1. Procédé de préparation de 1,4-butanediol, caractérisé en ce que l'on fait réagir du 2,3-dihydrofurane en une seule étape, en présence d'eau et d'hydrogène à une température de 20-300°C et sous une pression de 1-300 bar dans une gamme de pH de 2-7,5 en présence d'un catalyseur d'hydrogénation.

2. Procédé selon la revendication 1, caractérisé en ce que l'on utilise un catalyseur d'hydrogénation hétérogène.

3. Procédé selon la revendication 1 ou 2, caractérisé en ce que l'on utilise un catalyseur d'hydrogénation contenant au moins un élément choisi dans le groupe formé par les groupes Ib ou VIIb ou VIII de la classification périodique des éléments ou des mélanges de ces éléments.

4. Procédé selon l'une quelconque des revendications 1 à 3, caractérisé en ce que l'on utilise un catalyseur d'hydrogénation dont les composants à action catalytique sont déposés sur un support.

5. Procédé selon l'une quelconque des revendications 1 à 4, caractérisé en ce que l'on utilise un catalyseur d'hydrogénation contenant un ou plusieurs composants actifs acides de Brönsted ou de Lewis.

6. Procédé selon l'une quelconque des revendications 1 à 5, caractérisé en ce que l'on utilise un catalyseur d'hydrogénation contenant du rhénium.

7. Procédé selon l'une quelconque des revendications 1 à 5, caractérisé en ce que l'on utilise un catalyseur d'hydrogénation contenant du cuivre.

8. Procédé selon l'une quelconque des revendications 1 à 5, caractérisé en ce que l'on utilise un catalyseur d'hydrogénation contenant du ruthénium.

9. Procédé selon l'une quelconque des revendications 1 à 7, caractérisé en ce que le catalyseur contient au moins un élément des groupes Ib, VIIb ou VIII et, en outre, au moins un autre élément des groupes Ib, Vb, VIb, VIIb, VIII, IIIa et Va de la classification périodique des éléments, qui forme avec le ou les éléments des groupes Ib ou VIIb ou VIII un mélange ou un alliage.

10. Procédé selon l'une quelconque des revendications 1 à 8, caractérisé en ce que l'on utilise un catalyseur d'hydrogénation dont les composants à action catalytique sont déposés sur un support en oxyde d'aluminium, en alumine, en dioxyde de silicium, en dioxyde de zirconium, en dioxyde de titane, un zéolithe et/ou du charbon actif.

11. Procédé selon la revendication 1, caractérisé en ce que l'on utilise un catalyseur d'hydrogénation homogène contenant un élément du groupe VIII de la classification périodique des éléments.
